# EUROPEAN PATENT SPECIFICATION

(11) **EP 3 471 640 B1**
(45) Date of publication and mention of the grant of the patent: **22.07.2020**
(21) Application number: 17733328.3
(22) Date of filing: 19.06.2017
(51) Int. Cl.: A61B 18/14

(54) **HAND-HELD INSTRUMENT WITH BODY-SWIVEL**
HANDINSTRUMENT MIT KÖRPERDREHGELENK
INSTRUMENT PORTATIF PIVOTANT

(30) Priority: 17.06.2016 US 201662351809 P; 18.05.2017 US 201715599269
(43) Date of publication of application: 24.04.2019
(73) Proprietor: Megadyne Medical Products, Inc., Draper, UT 84020 (US)
(72) Inventor: GREEP, Darcy W., Draper, Utah 84020 (US); FRAMPTON, Chad S., Draper, Utah 84020 (US)
(74) Representative: Bettridge, Paul Sebastian
(86) International application number: PCT/US2017/038088
(87) International publication number: WO 2017/219012

(56) References cited:
- DE-U1-202015 103 912
- US-A1- 2014 276 763
- US-A1- 2015 209 071
- US-B1- 7 875 026

## Description

### BACKGROUND

### 1. Technical Field

This disclosure relates to hand-held instruments. More particularly, the disclosure relates to swivel components and functionality of hand-held instruments.

### 2. The Relevant Technology

As is known to those skilled in the art, modern surgical techniques typically employ radio frequency (RF) power to cut tissue and coagulate bleeding encountered in performing surgical procedures. For a historical perspective and details of such techniques, reference is made to United States Patent No. 4,936,842, issued to D'Amelio et al., and entitled "Electroprobe Apparatus".

US2015/209071 discloses an ultrasonice device for fingertip control, whereby an activation member and/or controller may be manipulated from various longitudinal positions on the handpiece and/or various rotational positions about the handpiece.

As is known to those skilled in the medical arts, electrosurgery is widely used and offers many advantages including the use of a single surgical instrument for both cutting and coagulation. A monopolar electrosurgical generator system has an active electrode, such as in the form of an electrosurgical instrument having a hand piece and a conductive electrode or tip, which is applied by the surgeon to the patient at the surgical site to perform surgery and a return electrode to connect the patient back to the generator.

The electrode or tip of the electrosurgical instrument is small at the point of contact with the patient to produce an RF current with a high current density in order to produce a surgical effect of cutting or coagulating tissue. The return electrode carries the same RF signal provided to the electrode or tip of the electrosurgical instrument, after it passes through the patient, thus providing a path back to the electrosurgical generator. To make the electrical connection for the RF current between the electrosurgical generator and the electrosurgical instrument, a cable having an electrically conductive core typically extends from the electrosurgical generator to the electrosurgical instrument.

Electrosurgical procedures often require precise movement and control of the electrosurgical instrument in order to properly treat the targeted tissue with the electrosurgical instrument. In particular, the manner in which the electrode tip is oriented and positioned relative to the targeted tissue can affect the way in which the tissue interacts with the delivered electrical energy.

In some instances, an operator may desire to readjust or reorient an electrosurgical instrument relative to the targeted tissue during an electrosurgical procedure. Using a typical electrosurgical instrument, such adjustments can increase the procedure time and typically require an operator to readjust his/her grip on the instrument, thereby increasing the risk of accidental contact between the instrument and non-targeted patient tissues.

In addition, moving and reorienting the electrosurgical instrument during a procedure typically requires moving the attached power cable and/or other hoses/connections as well. This leads to changes in the drag, torque, and torsional moment force distribution at the electrosurgical instrument, thereby altering the manner in which the instrument sits in the user's hand, making the instrument more difficult to consistently manipulate and control, and further increasing the risk of accident or procedural mistakes.

Further, changes in the way in which the instrument needs to be held or gripped as well as changes to the force distributions of the instrument against a user's hand can reduce user comfort during use of the instrument and can lead to faster hand fatigue.

The subject matter claimed herein is not limited to embodiments that solve any disadvantages or that operate only in environments such as those described above. Rather, this background is only provided to illustrate one exemplary technology area where some embodiments described herein may be practiced.

### BRIEF DESCRIPTION OF THE DRAWINGS

To further clarify the above and other advantages and features of the present invention, a more particular description of the invention will be rendered by reference to specific embodiments thereof which are illustrated in the appended drawings. It is appreciated that these drawings depict only illustrated embodiments of the invention and are therefore not to be considered limiting of its scope. The invention will be described and explained with additional specificity and detail through the use of the accompanying drawings in which:
Figure 1 illustrates an exemplary electrosurgical system;
Figure 2 illustrates an electrosurgical instrument as held by an operator;
Figures 3A-3B illustrate an electrosurgical instrument according to the present disclosure;
Figure 4 illustrates a cross-sectional view of the electrosurgical instrument of Figure 3, showing an extendable section positioned in a retracted position;
Figure 5 illustrates a cross-sectional view of the electrosurgical instrument of Figures 3 and 4, showing the extendable section positioned in an extended position; and
Figure 6 illustrates an electrosurgical instrument having a swivel section enabling reorientation of an electrode tip;
Figure 7 illustrates the electrosurgical instrument of Figure 6 with a portion of a hand piece removed to show an interior of the hand piece, and with a distal section in a first position;
Figure 8 illustrates the electrosurgical instrument of Figure 7 with a distal section swiveled to a second position;
Figure 9 illustrates the electrosurgical instrument of Figure 6 with a grip section removed to show a front piece of the electrosurgical instrument; and
Figure 10 illustrates the electrosurgical instrument of Figure 6 with the front piece removed to show a saddle section of the electrosurgical instrument.

### DETAILED DESCRIPTION

The invention is defined by the instrument of independent claim 1. Preferred embodiments are defined by the dependent claims. The present disclosure relates to electrosurgical instruments and other hand-held instruments having a swivel body enabling precision control and fine adjustment of the instrument during a procedure, such as during an electrosurgical procedure. In some embodiments, an electrosurgical or other hand-held instrument includes a hand piece having a proximal section and a distal section, the proximal section and distal section being rotationally decoupled to enable the distal section to be rotated independently of the proximal section, or vice versa.

One or more embodiments beneficially enable an electrosurgical instrument to be manipulated and reoriented without disrupting the grip position of the electrosurgical instrument in a user's hand. For example, during an electrosurgical procedure, a user may hold a hand piece by positioning a proximal section of the hand piece in the crook of his/her hand while gripping a distal section of the hand piece between the thumb and index and/or middle finger. The electrosurgical instrument enables the user to independently rotate the distal section relative to the proximal section, allowing the thumb and/or fingers to control the rotational manipulation of the distal section while the proximal section remains seated in the crook of the hand.

The structure and function of such embodiments can allow a user to adjust the electrosurgical instrument while minimizing or reducing changes in the force distribution (e.g., torque and drag effects) on the user's hand. Such benefits reduce or eliminate operator discomfort and fatigue, and help maintain consistent grip dynamics, thereby reducing or eliminating associated patient and equipment risks.

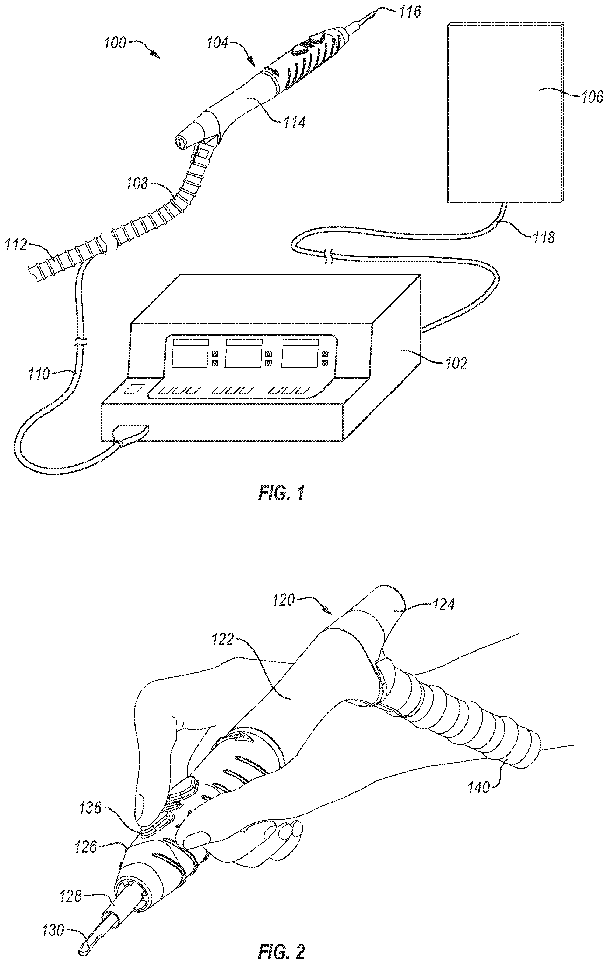

Figure 1 illustrates an exemplary electrosurgical system 100. The illustrated embodiment includes a signal generator 102, an electrosurgical instrument 104, and a return electrode 106. Generator 102, in one embodiment, is an RF wave generator that produces RF electrical energy. Connected to electrosurgical instrument 104 is a utility conduit 108. In the illustrated embodiment, utility conduit 108 includes a cable 110 that communicates electrical energy from generator 102 to electrosurgical instrument 104. The illustrated utility conduit 108 also includes a vacuum hose 112 that conveys captured/collected smoke and/or fluid away from a surgical site.

Generally, electrosurgical instrument 104 includes a hand piece or pencil 114 and an electrode tip 116. Electrosurgical instrument 104 communicates electrical energy to a target tissue of a patient to cut the tissue and/or cauterize blood vessels within and/or near the target tissue. Specifically, an electrical discharge is delivered from electrode tip 116 to the patient in order to cause heating of cellular matter of the patient that is in close contact with electrode tip 116. The tissue heating takes place at an appropriately high temperature to allow electrosurgical instrument 104 to be used to perform electrosurgery. Return electrode 106 is connected to generator 102 by a cable 118 in order to complete the circuit and provide a return electrical path to wave generator 102 for energy that passes into the patient's body.

As explained in greater detail below, some embodiments of electrosurgical or other hand-held instruments according to the present disclosure enable efficient capture of smoke generated during a procedure, such that smoke that is not immediately captured near the site of smoke generation (e.g., at the tissue/electrode tip interface) can still be captured and evacuated away from the operating environment. It will be appreciated, however, that smoke capture/evacuation is not required. Rather, for instance, some embodiments may include a hand piece with a swivel body as described herein but may not be equipped with smoke capture/evacuation features. Accordingly, even embodiments that are described and illustrated as including smoke capture/evacuation features are not so limited.

Illustrated in Figure 2 is an exemplary electrosurgical instrument 120 used to perform electrosurgical procedures and optionally evacuate smoke from a surgical site. Electrosurgical instrument 120 includes a hand piece 122 having a proximal end 124 and a distal end 126. An extendable section 128 is selectively extendable (e.g., translatable along an axis running in the proximal/distal direction) and includes a channel or conduit extending therethrough. An electrode tip 130 is received within the distal end of the extendable section 128. One or more power cables, one or more vacuum hoses, and/or other connections can be directed to the hand piece 122 through the utility conduit 140, which in the illustrated embodiment, is coupled to the hand piece 122 near the proximal end 124 and on an underside of the hand piece 122. Alternative embodiments can include utility conduit connections on a top and/or side section of a hand piece, at the proximal end extending proximally, or at other locations of the hand piece. The power cable communicates electrical energy from an electrosurgical generator to electrosurgical instrument 120. During an electrosurgical procedure, the electrical energy is passed through electrode tip 130 and into a patient's tissue.

Electrosurgical instruments, such as electrosurgical instrument 120, are commonly referred to as electrosurgical pencils or pens because in use they are often held in the same manner that a pencil or pen is held when writing. Figure 2 illustrates a common manner by which an operator can hold an electrosurgical instrument during an electrosurgical procedure. As shown, hand piece 122 is laid through the crook of the hand and is held in place by the middle finger and thumb. The index finger can be placed on top of hand piece 122 to further hold hand piece 122 in place as well as to control certain actions of the electrosurgical device through selective activation of one or more controls 136.

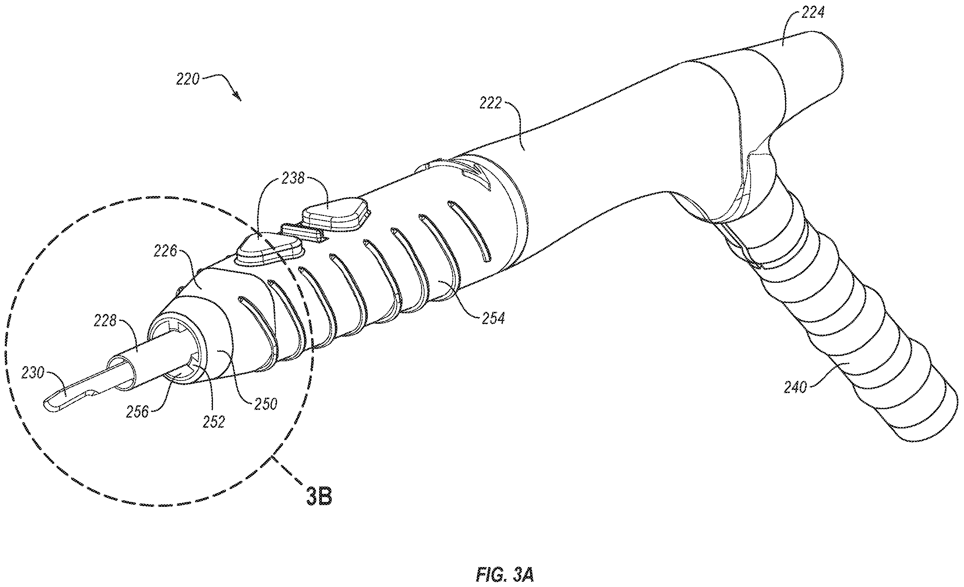

Figure 3A illustrates an embodiment of an electrosurgical instrument 220 configured with dual zone smoke evacuation. The illustrated embodiment includes a hand piece 222 having a proximal end 224 and a distal end 226. An extendable section 228 is disposed at least partially within the interior of the hand piece 222 and extends distally out of the hand piece 222. The extendable section 228 is configured to receive an electrode tip 230. In some embodiments, the extendable section 228 is formed from a conductive material and is configured to pass electrical current from a power cable (e.g., a power cable disposed within the illustrated utility conduit 240) to the electrode tip 230.

The extendable section 228 is preferably configured as a conduit (e.g., tube or other shape having a hollow or partially hollow cross-section). The conduit of the extendable section 228 is configured to pass at least partially into an internal chamber of the hand piece 222 and to be in fluid communication with the utility conduit 240 (and/or with a vacuum hose attached/disposed in the utility conduit 240). The extendable section 228 also includes a distal end opening providing fluid communication between the interior of the extendable section and the atmosphere exterior to the extendable section 228. As shown, the electrode tip 230 can be coupled to the extendable section 228 (e.g., via adhesive, welding, mechanical fastening, notches, slots, and/or friction fitting, or through integral formation of a single piece) in a manner that leaves one or more aperture spaces for smoke capture into the interior of the extendable section 228.

The illustrated embodiment can also include a front piece 250 having an opening for the extendable section 228 to pass through. In this embodiment, the front piece 250 is formed with a tapered profile that tapers inwardly in the distal direction. In other embodiments, the front piece 250 is formed without a tapered shape (e.g., a squared or straight edge profile), or with a different taper or curved profile.

The illustrated front piece 250 includes one or more supports 252 configured to hold or stabilize the extendable section 228 in position relative to the front piece 250. In some embodiments, the one or more supports 252 are configured to maintain the rotational relationship between the front piece 250 and the extendable section 228, such that rotation of the front piece 250 causes rotation of the extendable section 228 (and attached electrode tip 230) as well. Additionally, or alternatively, the extendable section 228 can be rotationally coupled to the front piece 250 and/or other rotatable portions of the hand piece 222 through other linking means, such as a key and keyway system, friction fitting system, or other configuration that maintains the translatability of the extendable section 228 while also rotationally coupling the extendable section 228 with the front piece 250.

The one or more supports 252 can be configured to frictionally maintain the position of the extendable section 228 relative to the front piece 250. For example, the one or more supports 252 can be configured to allow a user to adjust the extendable section 228 when desired, while otherwise maintaining the position of the extendable section 228 during normal operation of the instrument (e.g., securing against movement caused by gravity or other relatively minor forces).

The illustrated front piece 250 includes supports 252 formed as radial extensions. Other embodiments can include one or more legs, braces, helical extrusions, and/or other support structures. As shown, the front piece 250 includes an opening 256 to enable fluid communication between the atmosphere exterior to the hand piece 222 and the interior of the hand piece 222.

The embodiment shown in Figure 3A also includes a grip 254 configured to provide a tactile surface for a user to hold and/or control the electrosurgical instrument 220. The grip 254 can be formed from a rubber or polymer material, for example, and can include one or more ridges, grooves, and/or other surface features for providing comfort and/or tactile gripping enhancement to a user while holding the instrument. In addition, the rubber or polymer material may be of a thickness and material softness which improves the user grip on the hand piece 222, while being comformable to the user's fingers to provide a comfortable grip for both short and long term use.

The illustrated embodiment also includes one or more controls 238 enabling a user to adjust one or more parameters of the electrosurgical instrument 220, such as increasing or decreasing electrical power delivery through the instrument, turning the instrument on and off, adjusting the instrument for different operating modes (cut, coagulate, cut-coagulate blend), etc. For example, the controls 238 can provide a connection for transmitting control signals from the electrosurgical instrument 220 to an electrosurgical generator and/or other controller.

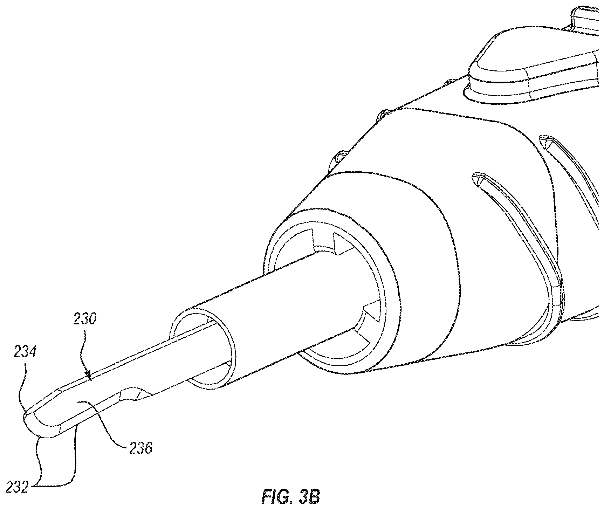

Figure 3B illustrates a closer view of the electrode tip 230. In the illustrated embodiment, the electrode tip 230 has a blade-like construction including an edge 232, point 234, and side faces 236. The blade-like formation allows a user to adjust the operative affect of the electrode tip 230 on a targeted tissue. For example, by positioning the edge 232 and/or point 234 of the electrode, which have a relatively small surface area, near the targeted tissue, the density of the current passing from the electrode tip 230 to the targeted tissue is distributed across a smaller area and is relatively higher (e.g., for use in a cut operation mode and/or pinpoint-type coagulation mode).

On the other hand, by rotating the electrode tip 230 relative to the targeted tissue to position a side face 236 of the electrode tip 230, which has a relatively higher surface area, near the targeted tissue, the density of the current passing from the electrode tip 230 to the targeted tissue is distributed across a greater area and is relatively lower (e.g., for use in a more dispersed spray-type coagulation mode). Rotation of the electrode tip 230 can therefore allow a user to perform different types of procedures and/or to dynamically adjust the operation of the electrosurgical instrument 220 during an electrosurgical procedure (e.g., by adjusting the level of pinpoint-type operation relative to spray-type operation and vice versa).

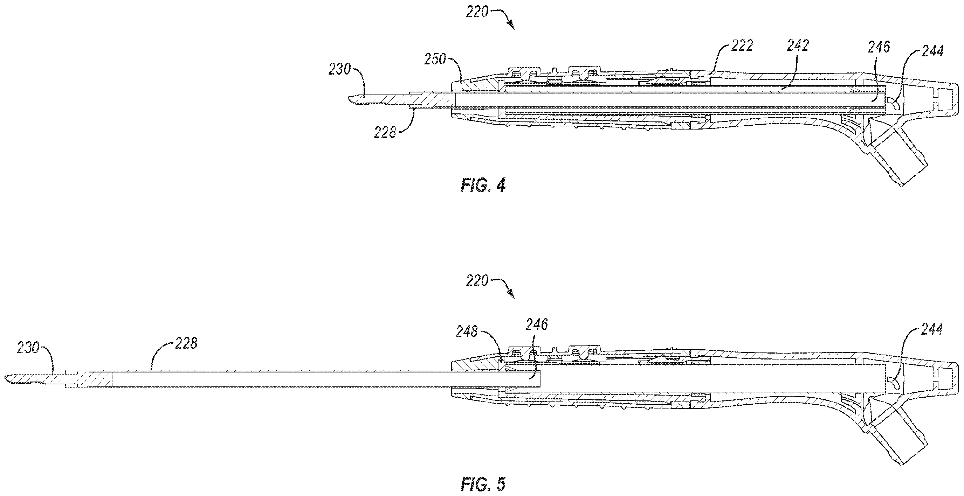

Figure 4 illustrates a cross-sectional view of the electrosurgical instrument 220 shown in Figure 3. Figure 4 illustrates the extendable section 228 in a retracted position, showing that much of the extendable section 228 can be positioned within the interior of the hand piece 222. The illustrated embodiment also includes an interior conduit 242 disposed within the interior of the hand piece 222 and configured in size and shape to enclose the extendable section 228 (e.g., at least the portions not extending distally beyond the hand piece 222) so that the extendable section 228 fits within the interior conduit 242 and is selectively translatable within the interior conduit 242.

In some embodiments, the electrosurgical instrument 220 includes a back stop 244 positioned to limit proximal translation of the extendable section 228 within the interior conduit 242. For example, the back stop 244 can be disposed at a position such that when the extendable section 228 is fully retracted, the electrode tip 230 is at or near the distal portion of the hand piece 222 but is not retracted into the interior of the hand piece 222. The illustrated back stop 244 is formed as a crosspiece to prevent proximal movement of the extendable section 228 past the back stop 244. Alternatively, the back stop 244 can be formed as a wall, rib, detent, abutment, catch, brace, and/or other means of preventing relative movement.

The illustrated electrosurgical instrument 220 also includes a connector 246 coupled to the extendable section 228 at the proximal end of the extendable section 228. As shown, the connector 246 has one or more projections extending radially outwardly to the wall of the interior conduit 242. In some embodiments, the one or more projections function as friction fitting components for maintaining the position of the extendable section 228 relative to the interior conduit 242 (e.g., in addition to, or as an alternative to, frictional securement through engagement between the supports 252 shown in figure 3A and the extendable section 228). For example, the connector 246 can be configured to hold the extendable section 228 in position during normal operation and movement of the electrosurgical instrument 220 (e.g., to hold position against gravity and/or against lighter forces resulting from movement of the hand piece 222) while still allowing user adjustment of the extendable section 228 under directed (e.g., hand-applied) force.

In some embodiments, the connector 246 is electrically conductive and is configured to pass electrical current to the extendable section 228. In some embodiments, the extendable section 228 is also electrically conductive and is able to pass electrical current to the electrode tip 230. For example, a power cable can extend into the interior of the hand piece 222 (e.g., through a utility conduit) to be coupled to the connector 246 and/or extendable section 228. Alternatively, the connector 246 and/or extendable section 228 can be formed of a non-conductive material, and a power cable or other conductive member can extend to the electrode tip 230 or to other intermediate components in order to deliver electrical current to the electrode tip 230. In some embodiments, the extendable section 228 and/or connector 246 may be formed from a conductive material that is at least partially coated with a non-conductive material to prevent the transfer of current from the extendable section 228 to patient tissue during an electrosurgical procedure.

As shown, the extendable section 228 can be formed with a length (measured along the proximal-distal axis) to be about the same length (e.g., within 99% of, 95% of, 90% of, 80% of, or 75%) of the hand piece 222 in which it can selectively translate within. In other embodiments, the extendable section 228 may be shorter or longer, such as about 0.75 times or 0.5 times the length of the hand piece 222, or about 1.25, 1.5, 2, or 2.5 times longer than the length of the hand piece 222.

Figure 5 illustrates the electrosurgical instrument 220 with the extendable section in an extended position. The embodiment shown in Figure 5 includes a front stop 248 configured to prevent distal movement of the extendable section 228 past the front stop 248. In this embodiment, the front stop 248 is configured as an annular structure having an inner diameter that is smaller than the inner diameter of the interior conduit 242. In this configuration, the extendable section 228 is prevented from passing further distally through the front stop 248 when the connector 246 is brought into contact with the front stop 248. For example, because the projections of the connector 246 extend to the wall of the interior conduit 242, they cannot pass through the smaller diameter of the front stop 248 and thereby limit the distal extension of the extendable section 228.

In other embodiments, movement of the extendable section 228 can be limited in other ways. For example, the extendable section 228 can include a key or keyway matched to a corresponding keyway or key of the hand piece 222 and/or front piece 250, and the key/keyway system can be formed to limit translation of the extendable section 228 to a desired range. In such embodiments, the key/keyway system can also function to lock the rotational relationship between the extendable section 228 and the front piece 250.

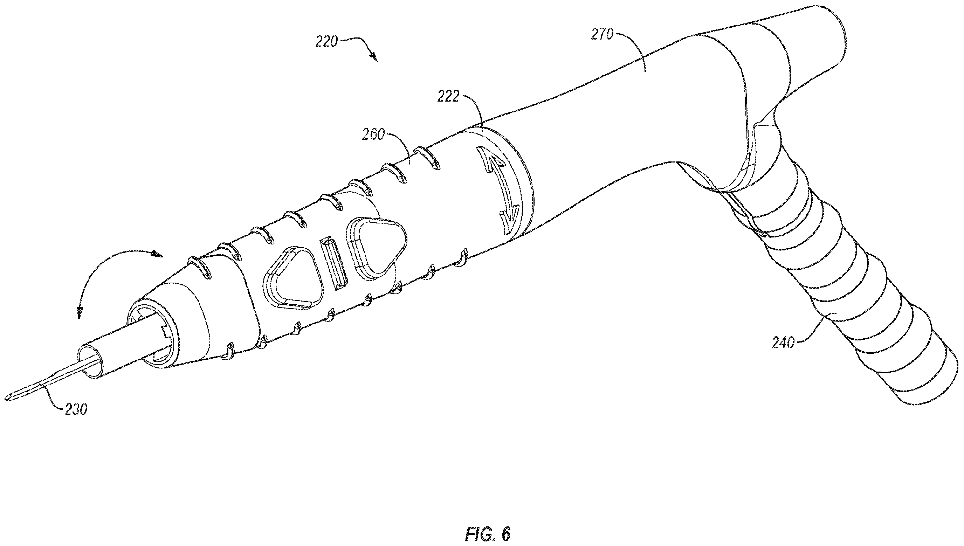

Figure 6 illustrates another view of the electrosurgical instrument 220, showing that the illustrated hand piece 222 includes a distal section 260 and a proximal section 270. As shown, the distal section 260 can be selectively rotated relative to the proximal section 270 (e.g., compare to the position shown in Figure 3). In the illustrated embodiment, the electrode tip 230 is configured to rotate with the distal section 260, allowing a user to adjust the angle of the electrode tip 230 by rotating the distal section 260.

For example, during an electrosurgical procedure, a user can rotate the distal section 260 to alter the orientation of the electrode tip 230 relative to a targeted tissue. This can beneficially enable a user to dynamically adjust the operational characteristics of the electrosurgical instrument, such as by altering the angle at which the electrode tip 230 interacts with the tissue (e.g., by adjusting which portion of the electrode is brought nearest the tissue). For example, the user can rotate the distal section 260 to angle the electrode edge nearer or farther from the target tissue, according to the user's preferences and/or patient needs. In addition, the electrosurgical instrument 220 allows a user to make dynamic adjustments during a procedure, such as by rotating the distal section 260 to adjust the angle of the electrode tip 230 to account for changing tissue geometries (e.g., curves, bumps, etc.) along a cutting or treatment path.

In a typical manner in which the hand piece 222 is held (see Figure 2, for example), the proximal section 270 is seated in the crook of the user's hand, while the distal section 260 is held between the user's thumb and middle finger and/or index finger. The hand piece 222 is configured to enable a user to make fine adjustments to the rotational position of the distal section 260 and electrode tip 230 using his/her thumb and/or fingers while the proximal section 270 remains seated within the crook of the user's hand. Such a configuration allows the desired adjustments to be made without changing the manner in which the hand piece 222 sits in the hand. This allows the user's grip position to be free from disruption during a rotational adjustment of the electrode tip 230. Enabling the grip position to be maintained can advantageously reduce accidents and patient risks associated with extraneous operator hand movements (e.g., inadvertently contacting the electrode with non-targeted tissue or sensitive equipment). In addition, reducing or eliminating the need to readjust the grip position prior to or following a rotational adjustment can shorten procedure time and reduce operator hand fatigue, further reducing associated risks to patients and equipment.

Further, by joining the utility conduit 240 to the proximal section 270, rotational movement of the distal section 260 is mechanically decoupled from the utility conduit 240, allowing rotational adjustments to be made without changing the force distribution on the hand piece 222 and without altering the drag, torque, or torsional moment forces resulting from connection of the utility conduit 240. This further allows the user's grip position to be maintained and provides more consistent controllability of the electrosurgical instrument 220 by keeping drag, torque, torsional moment forces, and other forces applied to the user's hand consistent throughout a procedure. For example, the utility conduit 240 can aid in anchoring the hand piece 222 in the user's hand in a stable manner, and by decoupling rotation of the distal section 260 from the proximal section 270 and utility conduit 240, this stable anchoring function can be maintained without swivel-induced fluctuation or change.

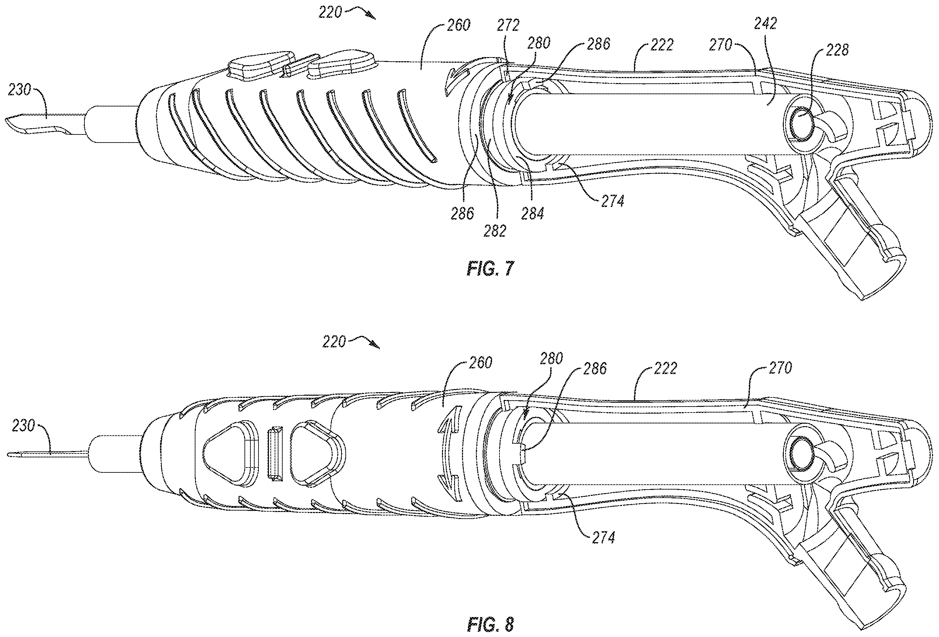

Figure 7 illustrates another view of the electrosurgical instrument 220 with a portion of the proximal section 270 removed in order to show internal components of the hand piece 222. From this view, the interior conduit 242 and the extendable section 228 can be seen extending from the distal section 260 to the proximal section 270 through an attachment piece 280. The attachment piece 280 is rotationally joined to the distal section 260 (e.g., rotation of the distal section 260 results in a corresponding rotation of the attachment piece 280), and is configured to couple the proximal section 270 to the distal section 260 while preserving the rotational independence of the respective components.

The illustrated attachment piece 280 is formed as a ring having a channeled section 282 and a rim 284 disposed proximal to the channeled section. The structure of the attachment piece 280 allows components of the instrument to be passed from the distal section 260 to the proximal section 270, and vice versa, through the opening of the ring structure. For example, this allows the extendable section 228 to be translatable within the interiors of both the distal section 260 and the proximal section 270.

In the illustrated embodiments, the channeled section 282 of the attachment piece 280 is disposed between the rim 284 and the proximal edge 286 of the front piece 250. As shown, the rim 284 and the proximal edge of the front piece 250 have diameters that are larger than the diameter of the attachment piece 280 at the channeled section 282. This enables the proximal section 270 to be linked to the distal section 260 through insertion of an inward radial extension 272 (disposed at the distal edge of the proximal section 270) into the channeled section 282 of the attachment piece 280, placing the extension 272 between the rim 284 and the proximal edge of the front piece 250. Proximal or distal separation of the distal section 260 from the proximal section 270 is therefore prevented, while independent rotational movement of the distal section relative to the proximal section is maintained.

The attachment piece 280 also includes a catch 286 projecting further proximally relative to the remaining proximal surface of the attachment piece 280. The proximal section 270 also includes a swivel stop 274 disposed at or near the proximal surface of the attachment piece 280. Rotation of the distal section 260 causes the attachment piece 280 to correspondingly rotate. Rotation can be continued until the catch 286 abuts against the swivel stop 274. The range of rotation can therefore be limited according to the position of the catch 286 and/or swivel stop 274.

Other devices omit swivel-limiting means, allowing a full 360 degree rotation of the distal section 260 relative to the proximal section 270. In some embodiments, rotation is limited to a range of about 45 to 315 degrees, or about 60 to 300 degrees, or about 90 to 270 degrees, for example.

Figure 8 illustrates a view of the hand piece with the distal section 260 in a rotated position relative to the view of Figure 7. As shown, rotation of the distal section 260 results in a corresponding rotation of the attachment piece 280, bringing the catch 286 closer to the swivel stop 274. Figure 8 also illustrates that the electrode tip 230 is correspondingly rotated with the distal section 260. As described herein, such rotation can enable an operator to adjust the orientation of the electrode tip 230 to a desired position, in order to provide different electrosurgical effects and/or to maintain a desired orientation during passage over rough or curving tissue geometries, for example.

In the embodiment illustrated in Figures 7 and 8, for example, Figure 7 shows the electrode tip 230 positioned with an edge of the blade-like structure aligned with the underside of the proximal section 270 (e.g., with the edge facing down). After rotation of the distal section 260 to the position shown in Figure 8, the electrode tip 230 is shown having a side face aligned with the underside of the proximal section 270 (e.g., with a side face facing down).

The illustrated embodiment provides a smooth interface between the channeled section 282 and the extension 272, allowing free rotation of the distal section 260 throughout the range of rotation. In other embodiments, rotation may be confined to discrete positions (e.g., in increments of 5, 10, 15, 20, 25, 30, 45, 60 degrees), such as by forming the grooved or sectioned interface between the channeled section 282 and the extension 272.

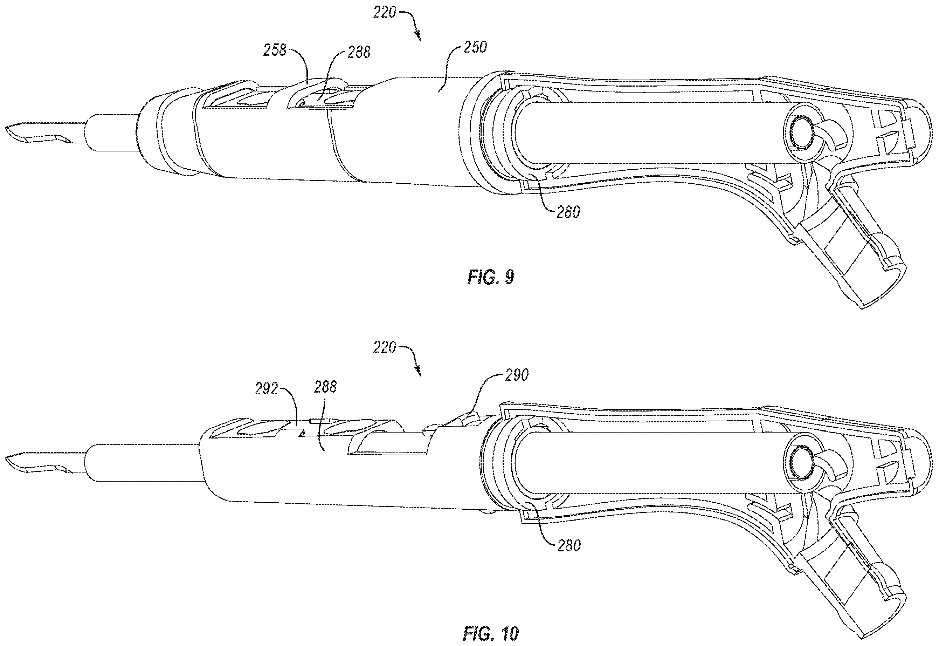

Figure 9 illustrates the electrosurgical instrument 220 without a grip. As shown, the front piece 250 at least partially encases a saddle piece 288 which, in this embodiment, is integrally joined to the attachment piece 280. The front piece 250 is configured to lock the rotation of the front piece 250 with the saddle piece 288 and attachment piece 280. For example, as shown in the illustrated embodiment, the front piece 250 can include a squared section 258 enabling the rotational association between the front piece 250 and the at least partially encased saddle piece 288, including the enjoined attachment piece 280.

Figure 10 illustrates the electrosurgical instrument 220 without a grip or front piece. As shown, the saddle piece 288 can be integrally joined to the attachment piece 280, such that rotation of the saddle piece 288 results in corresponding rotation of the attachment piece 280. In other embodiments, the attachment piece 280 can be coupled to the saddle piece 288 and/or front piece 250 through other means, such as through mechanical fastening, welding, adhesives, etc. The saddle piece 288 can be configured to engage with the front piece 250 through one or more connections, such as illustrated tab 290, and/or through a squared section 292 configured to match the squared section 258 of the front piece 250. In some embodiments, the saddle piece 288, attachment piece 280, and the front piece 250 are formed as one integral piece. In other embodiments, one or more of the listed components may be separately formed and coupled through other means (mechanical fastening, welding, adhesive bonding, friction fitting, etc.). In preferred embodiments, the front piece 250 and saddle piece 288 are separately formed.

While the embodiments described herein have been directed to electrosurgical instruments, the present disclosure is not intended to be so limited. Rather, the present disclosure is broadly directed to any hand-held instrument that includes a hand piece with a swivel body. Thus, for instance, a hand-held instrument according to the present disclosure need not include smoke capture/evacuation features. Similarly, a hand-held instrument for use in non-electrosurgical environments may include a functional implement other than an electrode tip for performing a desired function. Thus, reference herein to an electrode tip or tip is not limited to implements used to perform electrosurgical procedures. Rather, reference to an electrode tip or tip is intended to broadly refer to any functional implement that is or can be associated with a hand piece and which is usable to perform a desired function.

By way of non-limiting example, hand-held instruments according to the present disclosure may include dental instruments (e.g., drills, polishing tools, scalers, compressed air tools, suction tools, irrigation tools, carries detection tools, water flossing tool (e.g., waterpik)), soldering tools (e.g., heated tools, smoke collection tools, de-soldering tools), high speed grinding and polishing tools (e.g., Dremel tools, carving tools, manicure tools, dental lab grinders/polishers), laser treatment instruments, laser surgical instruments, light probes, suction handles (e.g., Yankauer), blasting tools (e.g., sandblast, gritblast), shockwave therapy tools, ultrasonic therapy tools, ultrasonic probe tools, ultrasonic surgical tools, adhesive application instruments, glue guns, pneumatic pipettes, welding tools, RF wrinkle therapy hand pieces, phaco hand pieces, shears, shaver, or razor hand pieces, micro drill hand pieces, vacuum hand pieces, small parts handling hand pieces, tattoo needle handles, small torch hand pieces, electrology hand pieces, low speed grinding, polishing and carving tools, permanent makeup hand pieces, electrical probe hand pieces, ferromagnetic surgical hand pieces, surgical plasma hand pieces, argon beam surgical hand pieces, surgical laser hand pieces, surgical suction instruments (e.g., liposuction cannulas), surgical suction cannulas, microdermabrasion hand pieces, fiberoptic camera handles, microcamera hand pieces, pH probe hand pieces, fiberoptic and LED light source hand pieces, hydrosurgery hand pieces, orthopedic shaver, cutter, burr hand pieces, wood burning tools, electric screwdrivers, electronic pad styluses, and the like.

The scope of the invention is defined by the appended claims.

## Claims

1. An hand-held instrument (220), comprising:
a hand piece (222) having a proximal section (270) and a distal section (260);
an attachment piece (280) configured to couple the distal section to the proximal section while enabling rotational independence of the distal section relative to the proximal section, and
a functional implement (230) extending distally from the distal section, the functional implement being rotationally linked with the distal section such that rotation of the distal section results in corresponding rotation of the functional implement, **characterised in that** the attachment piece includes a catch (286) projecting proximally from a proximal surface of the attachment piece, and wherein the proximal section includes a swivel stop (274) disposed so as to abut against the catch to limit rotation of the distal section.

2. The hand-held instrument of claim 1, wherein the attachment piece includes a channeled section, and wherein the proximal section includes an inward radial extension configured to insert into the channeled section to couple the proximal section to the attachment piece.

3. The hand-held instrument of claim 2, wherein the attachment piece includes a rim proximal of the channeled section, the proximal section extending distally over the rim and inserting into the channeled section.

4. The hand-held instrument of claim 2, wherein the distal section includes a front piece having a proximal edge disposed distally of the channeled section, the front piece having a diameter at the proximal edge that is larger than a diameter of the channeled section.

5. The hand-held instrument of claim 1, wherein the attachment piece is integrally joined to the distal section.

6. The hand-held instrument of claim 1, wherein the attachment piece is formed as a ring configured to allow passage of one or more conduits through an opening in the ring.

7. The hand-held instrument of claim 6, wherein at least one of the one or more conduits is an extendable section configured to be selectively translatable between an extended position and a retracted position.

8. The hand-held instrument of claim 1, wherein the catch and swivel stop are configured to provide a range of rotation of less than about 315 degrees of the distal section relative to the proximal section.

9. The hand-held instrument of claim 1, wherein the functional implement is an electrode tip configured to transmit electrical energy to target tissue.

10. The hand-held instrument of claim 9, wherein the electrode tip has an edge and a side face, and wherein the orientation of the edge and side face relative to a tissue surface is dynamically adjustable by rotation of the distal section.

11. The hand-held instrument of claim 1, the distal section includes a front piece at least partially encasing a saddle piece, the front piece having a squared section configured to engage with a squared section of the saddle piece to rotationally link the saddle piece to the front piece.

12. The hand-held instrument of claim 11, wherein:
the front piece is formed with a tapered profile that tapers inwardly in a distal direction; or
the attachment piece is integrally attached to the saddle piece.

13. The hand-held instrument of claim 1, further comprising a utility conduit attached to the proximal section, the utility conduit configured to maintain position relative to the proximal section when the distal section is rotated.

14. The hand-held instrument according to claim 7, wherein:
the extendable section is configured to be selectively translatable relative to the hand piece along a proximal/distal axis;
the functional implement comprises a tip extending distally from the extendable section;
the attachment piece is configured to enable rotation of the distal section about the proximal/distal axis while maintaining position of the proximal section; and
the distal section is configured to engage with the extendable section during rotation to rotationally link the extendable section and the tip with the distal section.

15. The hand-held instrument of claim 14, wherein the distal section includes one or more supports configured to engage with the extendable section to rotationally link the extendable section with the distal section.

## Patentansprüche

1. Handinstrument (220), umfassend:
ein Handteil (222) mit einem proximalen Abschnitt (270) und einem distalen Abschnitt (260),
ein Ansatzteil (280), das dazu ausgestaltet ist, den distalen Abschnitt an den proximalen Abschnitt zu koppeln, während es drehungsmäßige Unabhängigkeit des distalen Abschnitts bezüglich des proximalen Abschnitts ermöglicht, und
ein Funktionswerkzeug (230), das sich distal von dem distalen Abschnitt erstreckt, wobei das Funktionswerkzeug drehungsmäßig mit dem distalen Abschnitt verbunden ist, so dass eine Drehung des distalen Abschnitts zu einer entsprechenden Drehung des Funktionswerkzeugs führt,
**dadurch gekennzeichnet, dass** das Ansatzteil eine Raste (286) aufweist, die proximal von einer proximalen Fläche des Ansatzteils vorragt, und wobei der proximale Abschnitt einen Schwenkanschlag (274) aufweist, der so angeordnet ist, dass er an der Raste anliegt, um die Drehung des distalen Abschnitts begrenzt.

2. Handinstrument nach Anspruch 1, wobei das Ansatzteil einen Kanalabschnitt aufweist und wobei der proximale Abschnitt eine nach innen gehende radiale Verlängerung aufweist, die dazu ausgestaltet ist, sich in den Kanalabschnitt einzuschieben, um den proximalen Abschnitt an das Ansatzteil zu koppeln.

3. Handinstrument nach Anspruch 2, wobei das Ansatzteil einen proximal zu dem Kanalabschnitt liegenden Rand aufweist, wobei sich der proximale Abschnitt distal über den Rand erstreckt und sich in den Kanalabschnitt einschiebt.

4. Handinstrument nach Anspruch 2, wobei der distale Abschnitt ein Vorderteil mit einer proximalen Kante aufweist, die distal zu dem Kanalabschnitt angeordnet ist, wobei das Vorderteil einen Durchmesser an der proximalen Kante hat, der größer als ein Durchmesser des Kanalabschnitts ist.

5. Handinstrument nach Anspruch 1, wobei das Ansatzteil integral mit dem distalen Abschnitt verbunden ist.

6. Handinstrument nach Anspruch 1, wobei das Ansatzteil als ein Ring ausgebildet ist, der dazu ausgestaltet ist, den Durchgang einer oder mehrerer Leitungen durch eine Öffnung in dem Ring zu gestatten.

7. Handinstrument nach Anspruch 6, wobei mindestens eine der einen oder mehreren Leitungen ein ausfahrbarer Abschnitt ist, der dazu ausgestaltet ist, gezielt zwischen einer ausgefahrenen Position und einer zurückgezogenen Position translatierbar zu sein.

8. Handinstrument nach Anspruch 1, wobei die Raste und der Schwenkanschlag dazu ausgestaltet sind, einen Drehbereich des distalen Abschnitts bezüglich des proximalen Abschnitts von weniger als ungefähr 315 Grad bereitzustellen.

9. Handinstrument nach Anspruch 1, wobei das Funktionswerkzeug eine Elektrodenspitze ist, die zur Übertragung von elektrischer Energie an Zielgewebe ausgestaltet ist.

10. Handinstrument nach Anspruch 9, wobei die Elektrodenspitze eine Kante und eine Seitenfläche hat und wobei die Ausrichtung der Kante und der Seitenfläche bezüglich einer Gewebefläche durch Drehung des distalen Abschnitts dynamisch verstellbar ist.

11. Handinstrument nach Anspruch 1, wobei der distale Abschnitt ein Vorderteil aufweist, das ein Sattelteil mindestens teilweise umschließt, wobei das Vorderteil einen quadratischen Abschnitt hat, der dazu ausgestaltet ist, mit einem quadratischen Abschnitt des Sattelteils in Eingriff zu kommen, um das Sattelteil drehungsmäßig mit dem Vorderteil zu verbinden.

12. Handinstrument nach Anspruch 11, wobei:
das Vorderteil mit einem sich verjüngenden Profil ausgebildet ist, das sich nach innen in eine distale Richtung verjüngt, oder
das Ansatzteil ist integral an dem Sattelteil angebracht.

13. Handinstrument nach Anspruch 1, ferner umfassend eine an dem proximalen Abschnitt angebrachte Versorgungsleitung, wobei die Versorgungsleitung dazu ausgestaltet ist, bezüglich des proximalen Abschnitts an Ort und Stelle zu bleiben, wenn der distale Abschnitt gedreht wird.

14. Handinstrument nach Anspruch 7, wobei:
der ausfahrbare Abschnitt dazu ausgestaltet ist, gezielt bezüglich des Handteils entlang einer proximalen/distalen Achse translatierbar zu sein,
das Funktionswerkzeug eine Spitze umfasst, die sich distal von dem ausfahrbaren Abschnitt erstreckt,
das Ansatzteil dazu ausgestaltet ist, die Drehung des distalen Abschnitts um die proximale/distale Achse zu ermöglichen, während die Position des proximalen Abschnitts aufrechterhalten wird, und
der distale Abschnitt dazu ausgestaltet ist, während der Drehung mit dem ausfahrbaren Abschnitt in Eingriff zu kommen, um den ausfahrbaren Abschnitt und die Spitze drehungsmäßig mit dem distalen Abschnitt zu verbinden.

15. Handinstrument nach Anspruch 14, wobei der distale Abschnitt eine oder mehrere Stützen aufweist, die dazu ausgestaltet sind, mit dem ausfahrbaren Abschnitt in Eingriff zu kommen, um den ausfahrbaren Abschnitt drehungsmäßig mit dem distalen Abschnitt zu verbinden.

## Revendications

1. Instrument portatif (220), comprenant :
une pièce à main (222) ayant une section proximale (270) et une section distale (260) ;
une attache (280) conçue pour coupler la section distale à la section proximale tout en permettant l'indépendance en rotation de la section distale par rapport à la section proximale,
et un outil fonctionnel (230) s'étendant de façon distale à partir de la section distale, l'outil fonctionnel étant lié en rotation à la section distale de telle sorte que la rotation de la section distale entraîne une rotation correspondante de l'outil fonctionnel,
**caractérisé en ce que** l'attache comprend un cliquet (286) faisant saillie de manière proximale à partir d'une surface proximale de l'attache, et dans lequel la section proximale comprend une butée de pivotement (274) disposée de manière à venir en butée contre le cliquet pour limiter la rotation de la section distale.

2. Instrument portatif selon la revendication 1, dans lequel l'attache comprend une section canalisée, et dans lequel la section proximale comprend une extension radiale s'étendant vers l'intérieur conçue de manière à être insérée dans la section canalisée afin de coupler la section proximale à l'attache.

3. Instrument portatif selon la revendication 2, dans lequel l'attache comprend un rebord proximal de la section canalisée, la section proximale s'étendant distalement sur le rebord et s'insérant dans la section canalisée.

4. Instrument portatif selon la revendication 2, dans lequel la section distale comprend une pièce avant ayant un bord proximal disposé de manière distale par rapport à la section canalisée, la pièce avant ayant un diamètre au niveau du bord proximal qui est supérieur à un diamètre de la section canalisée.

5. Instrument portatif selon la revendication 1, dans lequel l'attache est assemblée intégralement à la section distale.

6. Instrument portatif selon la revendication 1, dans lequel l'attache est formée comme un anneau conçu pour permettre le passage d'un ou plusieurs conduits à travers une ouverture dans l'anneau.

7. Instrument portatif selon la revendication 6, dans lequel au moins l'un des conduits est une section extensible conçue pour pouvoir être sélectivement déplacée en translation entre une position étendue et une position rétractée.

8. Instrument portatif selon la revendication 1, dans lequel le cliquet et la butée de pivotement sont conçus pour fournir une plage de rotation inférieure à environ 315 degrés de la section distale par rapport à la section proximale.

9. Instrument portatif selon la revendication 1, dans lequel l'outil fonctionnel est une pointe d'électrode conçue pour transmettre l'énergie électrique au tissu cible.

10. Instrument portatif selon la revendication 9, dans lequel la pointe d'électrode a un bord et une face latérale, et dans lequel l'orientation du bord et de la face latérale par rapport à une surface de tissu est réglable de manière dynamique par rotation de la section distale.

11. Instrument portatif selon la revendication 1, la section distale comprend une pièce avant enveloppant au moins partiellement un élément étrier, la pièce avant ayant une section carrée conçue pour s'engager avec une section carrée de l'élément étrier pour lier en rotation l'élément étrier à la pièce avant.

12. Instrument portatif selon la revendication 11, dans lequel :
la pièce avant est formée d'un profil effilé qui se rétrécit vers l'intérieur dans une direction distale ; ou
l'attache est intégralement fixée à l'élément étrier.

13. Instrument portatif selon la revendication 1, comprenant en outre un conduit d'alimentation fixé à la section proximale, le conduit d'alimentation étant conçu pour maintenir la position par rapport à la section proximale lorsque la section distale est tournée.

14. Instrument portatif selon la revendication 7, dans lequel :
la section extensible est conçue de manière à pouvoir être sélectivement déplacée en translation par rapport à la pièce à main le long d'un axe proximal/distal ;
l'outil fonctionnel comprend une pointe s'étendant distalement à partir de la section extensible ;
l'attache est conçue pour permettre la rotation de la section distale autour de l'axe proximal/distal tout en maintenant la position de la section proximale ; et
la section distale est conçue pour s'engager avec la section extensible pendant la rotation afin de lier en rotation la section extensible et la pointe avec la section distale.

15. Instrument portatif selon la revendication 14, dans lequel la section distale comprend un ou plusieurs supports conçus pour s'engager avec la section extensible afin de lier en rotation la section extensible à la section distale.
